# EUROPEAN PATENT APPLICATION

(11) **EP 1 832 571 A1**
(43) Date of publication of application: **12.09.2007**
(21) Application number: 07003885.6
(22) Date of filing: 26.02.2007
(51) Int. Cl.: C07C 67/343, C07C 69/732

(54) **Process for producing hydroxyl group-containing vinyl compound**

(30) Priority: 27.02.2006 JP 2006050792
(71) Applicant: FUJIFILM Corporation, Minato-ku Tokyo (JP)
(72) Inventor: Shibuya, Akinori, Haibara-gun Shizuoka (JP)
(74) Representative: HOFFMANN EITLE

(57) **Abstract**

A process for producing a hydroxyl group-containing vinyl compound, the process comprising: reacting an acrylate compound and an aldehyde-based compound in a presence of a tertiary amine compound and water in a reaction solution to produce a hydroxyl group-containing vinyl compound, wherein the reaction is performed by adding an inorganic salt of a Group 1 or 2 metal of Periodic Table to the reaction solution.

## Description

### Background of the Invention

### 1. Field of the Invention

The present invention relates to a process for producing a hydroxyl group-containing vinyl compound by reacting an acrylate compound and an aldehyde-based compound.

The hydroxyl group-containing vinyl compound is widely used, for example, as a monomer for the production of a polymer assured of high refractive index and high heat resistance; a raw material for the production of various chemical products such as coating material, adhesive, detergent builder, resist material for semiconductors, and binder polymer for printing plates; or an intermediate of medical products such as anticancer drug and antiviral drug.

### 2. Description of the Related Art

Various methods for producing a hydroxyl group-containing vinyl compound have been heretofore proposed. For example, U.S. Patent 3,743,669 discloses a method of reacting a vinyl compound and an aldehyde-based compound in a liquid-phase uniform system at 0 to 200°C in the presence a cyclic tertiary amine catalyst. This reaction is generally known as Baylis-Hillman reaction, and a hydroxyl group-containing vinyl compound can be synthesized by a one-step reaction from a vinyl compound and an aldehyde-based compound. Therefore, many studies are being made on this reaction. However, the above-described method has a problem that the reaction rate is usually slow and moreover, the selectivity ratio of the hydroxyl group-containing vinyl compound is low, giving rise to a low reaction yield of the hydroxyl group-containing vinyl compound.

For the purpose of increasing the reaction rate, JP-A-8-301817 (the term "JP-A" as used herein means an "unexamined published Japanese patent application") discloses a technique where at the time of producing a hydroxyl group-containing vinyl compound through a reaction between an acrylate compound and an aldehyde-based compound, the reaction is performed by using a tertiary amine catalyst, DABCO, in an organic solvent capable of dissolving 5 mass% or more of water and having added thereto water over 0.5 to 2.0 times the maximum amount of water that can be contained in the reaction solution without causing two-phase separation at the initiation of reaction. The reaction rate becomes higher by this addition of water than that in conventional methods but is not yet satisfied. Also, there is a problem that an ether dimer produced resulting from dehydration by two molecules of the objective hydroxyl group-containing vinyl compound and impurities having a boiling point close to that of the objective are produced in a large amount and the purification incurs a great load.

Furthermore, for the purpose of overcoming the slow reaction rate out of the above-described problems, JP-A-7-285906 has proposed a production process where at the time of producing a hydroxyl group-containing vinyl compound by reacting an acrylate compound and an aldehyde-based compound, the reaction is performed by using a tertiary amine compound having a specific N-methyl group as the catalyst in the presence of water in an amount large enough to form an aqueous phase at the completion of reaction. In this production process, the reaction rate can be significantly increased as compared with conventional methods and the objective hydroxyl group-containing vinyl compound can be obtained at a high yield. However, it has been found that in this production process, the raw material acrylate compound is partially hydrolyzed due to strong basicity of the tertiary amine compound used as the catalyst and the catalytic activity is sometimes reduced. Thus, there is room for improvement. As an improvement measure therefor, JP-A-2001-302586 discloses a technique where a specific aprotic polar solvent of giving a uniform system at the completion of reaction is caused to be present and the hydrolysis is thereby suppressed, but more improvement is demanded.

### Summary of the Invention

Accordingly, an object of the present invention is to provide a process for producing a hydroxyl group-containing vinyl compound by reacting an acrylate compound and an aldehyde-based compound in the presence of a tertiary amine compound catalyst and water, where a high reaction rate and a high reaction yield are achieved and the production of by-products is suppressed.

As a result of intensive studies on the compounds showing catalytic activity of enhancing the reaction rate in this reaction system, the present inventors have found that when a specific inorganic salt is added to the reaction solution, not only the reaction rate is increased and the objective compound can be obtained at a high reaction yield but also the production of by-products can be suppressed.

That is, the present invention is as follows.
(1) A process for producing a hydroxyl group-containing vinyl compound, the process comprising:
   reacting an acrylate compound and an aldehyde-based compound in a presence of a tertiary amine compound and water in a reaction solution to produce a hydroxyl group-containing vinyl compound,
   wherein the reaction is performed by adding an inorganic salt of a Group 1 or 2 metal of Periodic Table to the reaction solution.
(2) The process for producing a hydroxyl group-containing vinyl compound as described in (1) above,
   wherein the inorganic salt is a lithium salt, a sodium salt or a potassium salt.
(3) The process for producing a hydroxyl group-containing vinyl compound as described in (1) or (2) above,
   wherein a molar ratio (inorganic salt/acrylate compound) between the inorganic salt and the acrylate compound is from 0.2 to 10.
(4) The process for producing a hydroxyl group-containing vinyl compound as described in any of (1) to (3) above,
   wherein a molar ratio (acrylate compound/aldehyde-based compound) between the acrylate compound and the aldehyde-based compound is 2 or less.
(5) The process for producing a hydroxyl group-containing vinyl compound as described in any of (1) to (4) above,
   wherein a molar ratio (water/aldehyde-based compound) between the water and the aldehyde-based compound is from 7 to 20.
(6) The process for producing a hydroxyl group-containing vinyl compound as described in any of (1) to (5) above,
   wherein the aldehyde-based compound is paraformaldehyde.
(7) The process for producing a hydroxyl group-containing vinyl compound as described in any of (1) to (6) above,
   wherein the reaction is performed by further adding to the reaction solution an aprotic polar solvent in an amount not allowing the reaction solution to form a two-phase system at initiation of the reaction.
(8) The process for producing a hydroxyl group-containing vinyl compound as described in any of (1) to (7) above,
   wherein the inorganic salt is added to the reaction solution before the reaction starts.

According to the present invention, an inorganic salt of a specific metal is used as a compound showing catalytic activity of enhancing the reaction rate, so that a production process for a hydroxyl group-containing vinyl compound, where high reaction rate and high reaction yield are achieved and the production of by-products is suppressed, can be provided.

### Detailed Description of the Invention

The present invention is further described below.

The hydroxyl group-containing vinyl compound produced according to the present invention is not particularly limited, but examples thereof include a compound represented by the following formula (1): 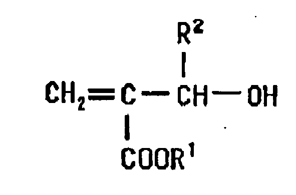 (wherein R¹ represents an organic residue, and R² represents a hydrogen atom or an organic residue).

Specific examples thereof include alkyl 2- (hydroxymethyl)acrylates such as methyl 2-(hydroxymethyl)acrylate, ethyl 2-(hydroxymethyl)acrylate, n-butyl 2-(hydroxymethyl)acrylate, tert-butyl 2-(hydroxymethyl)acrylate, 2-ethylhexyl 2-(hydroxymethyl)acrylate, cyclohexyl 2-(hydroxymethyl)acrylate; and alkyl 2-(1-hydroxy-1-alkylmethyl)acrylates such as methyl 2-(1-hydroxyethyl)-acrylate, ethyl 2-(1-hydroxyethyl)acrylate, n-butyl 2-(1-hydroxyethyl)acrylate, methyl 2-(1-hydroxybutyl)acrylate, ethyl 2-(1-hydroxybutyl) acrylate, n-butyl 2-(1-hydroxybutyl)acrylate, methyl 2-(1-hydroxybenzyl)acrylate, ethyl 2-(1-hydroxybenzyl)acrylate and n-butyl 2-(1-hydroxybenzyl)acrylate. In particular, the present invention is suitable for the production of methyl 2-(hydroxymethyl)-acrylate, ethyl 2-(hydroxymethyl)acrylate, n-butyl 2-(hydroxymethyl)acrylate, tert-butyl 2-(hydroxymethyl)-acrylate, 2-ethylhexyl 2-(hydroxymethyl)acrylate and cyclohexyl 2-(hydroxymethyl)acrylate.

The acrylate compound used as a raw material in the present invention is a compound represented by the following formula (2):

**CH₂=CH-COOR¹**

(wherein R¹ represents an organic residue, preferably an alkyl group..having a carbon number of 1 to 18, a cycloalkyl group having a carbon number of 3 to 10, an aryl group, a hydroxyalkyl group having a carbon number of 1 to 8, a -(CH₂)ₘNR³R⁴ group, a -(CH₂)ₙN⁺R⁵R⁶R⁷·M⁻ group or a -(C₂H₄O)ₒR⁸ group (wherein the substituents R³, R⁴, R⁵, R⁶, R⁷ and R⁸ each is independently a linear or branched alkyl group having a carbon number of 1 to 8, m and n each is an integer of 2 to 5, the anion M⁻ is Cl⁻, Br⁻, CH₃COO⁻, HCOO⁻, SO₄²⁻ or PO₄³⁻, and o is an integer of 1 to 80)).

Specific examples of the acrylate compound include:
(a) an acrylate where the substituent is an alkyl group having a carbon number of 1 to 18, such as methyl acrylate, ethyl acrylate, n-propyl acrylate, isopropyl acrylate, n-butyl acrylate, isobutyl acrylate, tert-butyl acrylate, n-octyl acrylate, isooctyl acrylate, 2-ethylhexyl acrylate, lauryl acrylate and stearyl acrylate;
(b) a cycloalkyl acrylate where the substituent is a cycloalkyl group having a carbon number of 3 to 10, such as cyclopentyl acrylate, cyclohexyl acrylate and cyclohexylmethyl acrylate;
(c) an aryl acrylate where the substituent is an aryl, such as phenyl acrylate, o-methoxyphenyl acrylate, p-methoxyphenyl acrylate and benzyl acrylate;
(d) a hydroxyalkyl acrylate where the substituent is a hydroxyalkyl group having a carbon number of 1 to 8, such as 2-hydroxyethyl acrylate, 2-hydroxypropyl acrylate, 3-hydroxypropyl acrylate and 4-hydroxybutyl acrylate;
(e) an alkylamino acrylate where the substituent is a -(CH₂)ₘNR³R⁴ group, such as N,N-dimethylaminoethyl acrylate, N,N-diethylaminoethyl acrylate, N,N-dimethylaminopropyl acrylate, N,N-dimethylaminobutyl acrylate, N,N-diethylaminobutyl acrylate and N,N-dimethylaminopentyl acrylate;
(f) a quaternary ammonium compound of an alkylamino acrylate where the substituent is a -(CH₂)ₙN⁺R⁵R⁶R⁷·M⁻ group, such as quaternary ammonium compound of N,N-dialkylamino acrylate; and
(g) acrylates where the substituent is a -(C₂H₄O)ₒR⁸ group, such as methoxyethyl acrylate, ethoxyethyl acrylate, lauryloxytrioxyethyl acrylate, and methoxypolyoxyethylene acrylate with o being from 1 to 80, preferably from 1 to 30. Among these acrylates, preferred are methyl acrylate, ethyl acrylate, n-butyl acrylate, 2-ethylhexyl acrylate, 2-hydroxyethyl acrylate and 2-hydroxypropyl acrylate.

Examples of the aldehyde-based compound for use in the present invention include an aldehyde group-containing compound; trioxane, paraformaldehyde, paraacetaldehyde; an aldehyde compound represented by the following formula (3):

**Y-CHO**

(wherein Y represents a hydrogen atom or a linear or branched alkyl group having a carbon number of 1 to 8); and an oxymethylene compound represented by the following formula (4): 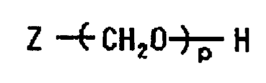 (wherein Z represents a hydrogen atom, a linear or branched alkyl group having a carbon number of 1 to 8, or a cycloalkyl group having a carbon number of 3 to 10, and p represents an integer of 1 to 100).

Paraformaldehyde, trioxane and the like are a compound resulting from the condensation of formaldehyde, and such a compound undergoes separation in water and acts like formaldehyde.

Examples of the aldehyde group-containing compound include formaldehyde, acetaldehyde, propionaldehyde, butylaldehyde, valeraldehyde, isobutylaldehyde, pivalinaldehyde, cyclohexylaldehyde, cyclohexenealdehyde, benzaldehyde and furfural. Specific examples of the oxymethylene compound include paraformaldehyde, a 20 to 50 mass% aqueous solution of formaldehyde (formaldehyde hydrate), and a methanol aqueous solution having a formaldehyde concentration of 20 to 50 mass%. Among these aldehyde-based compounds, paraformaldehyde is particularly suitable. The paraformaldehyde is a polymer (8- to 100-mer) of formaldehyde, and this is a solid matter having a granular or powder shape at ordinary temperature. Industrially available paraformaldehyde usually contains moisture, and the water content may be 20 mass% or less. One species of those aldehyde-based compounds may be used alone or when the present invention is industrially implemented, considering the easy handling or the like of the aldehyde-based compound, two or more species thereof may be appropriately mixed and used.

The amount of the acrylate-based compound used with respect to the aldehyde-based compound may be selected such that the molar ratio (acrylate compound/aldehyde-based compound) between the acrylate compound and the aldehyde-based compound becomes 2 or less, preferably 1 or less, more preferably 0.25 to 0.75. If the molar ratio exceeds 2, a large amount of the acrylate compound remains in the reaction system to decrease the purity of the objective compound and therefore, a cumbersome purification process such as column chromatography is disadvantageously required.

Examples of the tertiary amine compound used as the catalyst in the present invention include trimethylamine, triethylamine, tri-n-propylamine, triisopropylamine, tributylamine, triisobutylamine, tri-n-pentylamine, N-methyldiisopropylamine, N,N-diethylisopropylamine, N,N-dimethylethylamine, N,N-dimethylisopropylamine, tri-2-ethylhexylamine, N-methyldiethylamine, N,N-dimethyl-n-propylamine, N,N-dimethyl-n-butylamine, N,N-dimethylisobutylamine, N,N-dimethyl-(2-ethylhexyl)amine, N,N-diisopropyl-(2-ethylhexyl)amine, N,N-di-n-butyl-(2-ethylhexyl)amine, N-methyl-di(2-ethylhexyl)amine, N-n-butyl-di(2-ethylhexyl)amine, N-isobutyl-di(2-ethylhexyl)-amine, pyrrocoline, quinolidine, 1,4-diazabicyclo-[2,2,2]-octane and hexamethylenetetramine. Among these, 1,4-diazabicyclo-[2,2,2]-octane (DABCO) and hexamethylenetetramine are preferred.

One species of these tertiary amine compounds may be used alone, or two or more species thereof may be appropriately mixed and used. The tertiary amine compound can be used in various states such as liquid and gas. The amount of the tertiary amine compound used is not particularly limited, but the ratio (by mol) of tertiary amine compound/aldehyde-based compound is usually from 0.05 to 2, preferably from 0.3 to 1.0. By using the tertiary amine compound at a molar ratio within the range of 0.3 to 1.0, a high reaction rate can be maintained and at the same time, the objective hydroxyl group-containing vinyl compound can be obtained at a high selectivity ratio.

In the present invention, an aprotic polar solvent is preferably added in the co-presence of the above-described tertiary amine compound and water, because the reaction rate can be more increased.

The amount of water used is determined to give, in combination with the aprotic polar solvent added, a uniform system at the initial stage of reaction (preferably also at the completion of reaction). This amount varies depending on, for example, the combination with the aprotic polar solvent or the kind of the acrylate compound used and therefore, its detailed range can be hardly limited, but the amount used may be selected such that the molar ratio (water/aldehyde-based compound) between water and the aldehyde-based compound becomes preferably 7 or more, more preferably from 7 to 20, still more preferably from 10 to 20. If the molar ratio is less than 10, a dimer by-product derived from the objective hydroxyl group-containing vinyl compound is produced in a large amount due to the high reaction concentration, whereas if the molar ratio exceeds 20, the aprotic polar solvent needs to be added in a large amount so as to give a uniform system at the completion of reaction and great labor is sometimes required for the separation or recovery of the aprotic polar solvent. The amount of water as referred to herein means the total amount of water present in the reaction system and as for the method of adding water, a necessary amount of water may be added independently or the amount of water may be adjusted using a solution obtained by previously adding a necessary amount of water to the tertiary amine compound or aldehyde compound.

The aprotic polar solvent used for the reaction of the present invention is not particularly limited but is preferably a nitrogen atom-containing aprotic polar solvent such as compounds except for the tertiary amine compound used above as the catalyst, more preferably a tertiary amine compound not having an active hydrogen. Specific examples thereof include a tertiary amine compound such as tetrahydrofuran, pyridine, methylpyridine, α-picoline, β-picoline, γ-picoline, N,N-dimethylaniline, N,N-diethylaniline, N-methylpiperidine, N-ethypiperidine, N-methylpiperazine, N-ethylpiperazine, N-methylmorpholine, N-ethylmorpholine, N,N,N',N'-tetramethyldiaminoethane and N,N,N',N'-tetramethyldiaminopropane; and an amide compound such as N,N-dimethylformamide, N,N,-diethylformamide, N,N-dimethylacetamide, N,N-diethylacetamide, N-methylpyrrolidone and N-ethylpyrrolidone. In particular, those having a boiling point of 30 to 250°C at atmospheric pressure and being easily recoverable by distillation are preferred, and N-methylmorpholine, N-ethylmorpholine, N,N-dimethylformamide (DMF), N,N-dimethylacetamide (DMAc) and N-methylpyrolidone (NMP), which are a nitrogen-containing aprotic polar solvent, are more preferred.

The amount of the aprotic polar solvent added is appropriately determined, in combination with the amounts of water and acrylate compound used and the kind of aprotic polar solvent added, to prevent formation of a two-phase system, that is, to give a uniform system, in the reaction solution at the initial stage of reaction, but the amount added may be selected preferably to ensure a uniform reaction system from the initiation of reaction to the completion of reaction, more preferably to give a molar ratio (aprotic polar solvent/(water + acrylate compound)) between the aprotic polar solvent and (water + acrylate compound) of 0.2 to 1.0, still more preferably from 0.2 to 0.7.

As for the reaction concentration in the present invention, the concentration of the acrylate compound based on the amount of the reaction solvent comprising water or water/aprotic polar solvent is preferably from 0.2 to 0.70 mol/liter, more preferably from 0.3 to 0.6 mol/liter. If the reaction concentration is less than 0.2 mol/liter, a low yield of the objective compound with respect to the reaction vessel, that is, decrease in the productivity is incurred due to the low reaction concentration, whereas if it exceeds 0.70 mol/liter, by-products such as ether dimer resulting from dehydration using two molecules of the hydroxyl group-containing vinyl compound are produced in a large amount and great labor is sometimes required for the separation of by-products.

The inorganic salt of a Group 1 or 2 metal of Periodic Table, which is an essential component of the present invention, is described below. Incidentally, the metals belonging to Groups 1 and 2 of Periodic Table indicate an alkali metal and an alkaline earth metal.

In the process of the present invention, an inorganic salt of a Group 1 or 2 metal of Periodic Table is added, whereby a cation of a Group 1 or 2 metal of Periodic Table comes to be present in the reaction solution having the above-described water/reaction solvent system.

The inorganic salt is preferably added before the reaction is started by the temperature elevation.

The inorganic salt of a Group 1 or 2 metal of Periodic Table is not particularly limited as long as it is a compound dissolvable in the water/reaction system, but specific examples thereof include lithium chloride, sodium chloride, potassium chloride, lithium bromide, sodium bromide, potassium bromide, lithium iodide, sodium iodide, potassium iodide, calcium chloride, lithium fluoride, sodium fluoride, potassium fluoride, lithium tetrafluoroborate and sodium hexafluorophosphate. In particular, a lithium salt, a sodium salt and a potassium salt are preferred and in view of general-purpose use, lithium chloride and sodium chloride are more preferred. The amount of the inorganic salt used may be selected such that the molar ratio (inorganic salt/acrylate compound) between the inorganic salt and the acrylate compound becomes from 0.2 to 10, preferably from 0.5 to 5.0.

The acrylate compound used for the reaction and the objective hydroxyl group-containing vinyl compound each has a property of being readily polymerized. Therefore, a polymerization retarder (or polymerization inhibitor) or a molecular oxygen is preferably added to the reaction system so as to suppress the polymerization during the reaction.

Examples of the polymerization retarder include, but are not limited to, quinones such as hydroquinone, methylhydroquinone, tert-butylhydroquinone, 2,4-di-tert-butylhydroquinone, 2,4-dimethylhydroquinone; an amine compound such as phenothiazine; phenols such as 2,4-dimethyl-6-tert-butylphenol, 2,4-di-tert-butylphenol and p-methoxyphenol; substituted catechols such as p-tert-butylcatechol; and substituted resorcins. One species of these polymerization retarders may be used alone, or two or more species thereof may be appropriately mixed and used.

The amount of the polymerization retarder added is not particularly limited but may be selected, for example, such that the ratio to the acrylate compound becomes from 0.01 to 1 mass%. As for the molecular oxygen, for example, air or a mixed gas of molecular oxygen and nitrogen may be used. In this case, the gas containing molecular oxygen may be blown into the reaction solution. In order to satisfactorily suppress the above-described polymerization, it is preferred to use a polymerization retarder and a molecular oxygen in combination.

The reaction temperature in the reaction according to the present invention is not particularly limited but in order to suppress the polymerization as above, the reaction temperature is preferably from 30 to 150°C, more preferably from 60 to 80°C. If the reaction temperature is less than 30°C, a slow reaction rate and an excessively long reaction time result and this is not preferred for industrial production of the hydroxyl group-containing vinyl compound, whereas if the reaction temperature exceeds 150°C, the above-described polymerization is disadvantageously difficult to suppress.

The reaction time in the reaction according to the present invention may be appropriately set to complete the reaction and is not particularly limited, but in general, a reaction time of approximately from 1 to 7 hours is sufficient and this is shorter than that in conventional methods. Also, the reaction pressure is not particularly limited and may be any of ordinary pressure (atmospheric pressure), reduced pressure and applied pressure.

After the completion of reaction, the reaction solution is subjected as it is to fractional distillation or after neutralizing the tertiary amine compound by adding an acidic compound to the reaction solution, the fractional distillation is performed, whereby the objective hydroxyl group-containing vinyl compound can be recovered.

### [Examples]

The present invention is described in greater detail below by referring to Examples.

Incidentally, the identification of compound was performed by ¹H-NMR and IR. Also, the quantitative determination of compound was performed by high-performance liquid chromatography (HPLC).

### [Example 1]

In a 1,000 ml-volume four neck-flask equipped with a thermometer, a gas blowing tube, a condenser tube, a stirring device and a water bath, 324 ml (18 mol) of additive water, 756 ml (8.7 mol) of N,N-dimethylacetamide, 91.6 g (2.16 mol) of lithium chloride and 0.1 g of p-methoxyphenol as a polymerization retarder were added to 54 g (0.54 mol) of ethyl acrylate, 34.1 g (1.1 mol) of paraformaldehyde and 72.9 g (0.65 mol) of DABCO, and the reaction solution was stirred at 50°C for 4 hours, thereby allowing the reaction to proceed. This reaction proceeded in a uniform system from the initiation of reaction to the completion of reaction.

After the completion of reaction, the reaction solution was allowed to cool to an inner temperature of 30°C, then cooled to 10°C or less by using an ice bath and after charging 650 ml of 4 N hydrochloric acid, further stirred for 15 minutes. The organic layer separated by extraction with ethyl acetate was washed with 1,080 ml of distilled water and further washed with an aqueous 5% sodium hydrogencarbonate solution. This organic layer was dried over magnesium sulfate and after adding p-methoxyphenol to account for 600 ppm, concentrated by a rotary evaporator, as a result, the objective ethyl 2-(hydroxymethyl) acrylate was obtained and this was confirmed by ¹H-NMR.

The obtained compound was analyzed by high-performance liquid chromatography (HPLC) and it was confirmed that ethyl 2-(hydroxymethyl)acrylate was produced in a yield of 88% based on the charged acrylate compound. Incidentally, in the analysis by HPLC, the measurement was performed at a wavelength of 210 nm.

### [Examples 2 to 16]

The process was performed under the same conditions except for changing the starting acrylate compound, solvent added (aprotic polar solvent), tertiary amine compound catalyst, inorganic salt and reaction time in Example 1. The yields of the objective compounds are shown together with the reaction conditions in Table 1.

### [Comparative Example 1]

The process was performed under the same conditions except for not adding lithium chloride in Example 1. The results are shown in Table 1.

**Table 1**

| Example | Acrylate | Aldehyde | Tertiary Amine | Pure Water | Solvent | Inorganic Salt | Temperature | Time | Yield |
|---|---|---|---|---|---|---|---|---|---|
| 1 | ethyl acrylate (0.54 mol) | paraformaldehyde (1.1 mol) | DABCO (0.65 mol) | 324 ml (18 mol) | DMAc (8.7 mol) | lithium chloride (2.16 mol) | 50°C | 4 hours | 88% |
| 2 | methyl acrylate (0.54 mol) | paraformaldehyde (1.1 mol) | DABCO (0.65 mol) | 360 ml (20 mol) | DMAc (8.7 mol) | lithium chloride (2.16 mol) | 50°C | 4 hours | 87% |
| 3 | cyclohexyl acrylate (0.54 mol) | paraformaldehyde (1.1 mol) | DABCO (0.65 mol) | 324 ml (18 mol) | DMAC (8.7 mol) | lithium chloride (2.16 mol) | 50°C | 4 hours | 90% |
| 4 | cyclohexyl acrylate (0.54 mol) | 35% methanol aqueous solution of formaldehyde (0.9 mol) | DABCO (0.65 mol) | 324 ml (18 mol) | DMAc (8.7 mol) | lithium chloride (2.16 mol) | 50°C | 5 hours | 82% |
| 5 | ethyl acrylate (0.54 mol) | paraformaldehyde (1.1 mol) | aqueous 30% trimethylamine solution (0.50 mol) | 324 ml (18 mol) | DMAc (8.7 mol) | lithium chloride (2.16 mol) | 50°C | 4 hours | 88% |
| 6 | cyclohexyl acrylate (0.54 mol) | paraformaldehyde (1.1 mol) | DABCO (0.65 mol) | 324 ml (18 mol) | NMP (9.5 mol) | lithium chloride (2.16 mol) | 50°C | 4 hours | 90% |
| 7 | cyclohexyl acrylate (0.54 mol) | paraformaldehyde (1.1 mol) | DABCO (0.80 mol) | 360 ml (20 mol) | DMF (9.0 mol) | lithium chloride (2.16 mol) | 50°C | 4.5 hours | 86% |
| 8 | cyclohexyl acrylate (0.54 mol) | paraformaldehyde (1.1 mol) | DABCO (0.65 mol) | 324 ml (18 mol) | Acetonitrile (10 mol) | lithium chloride (1.0 mol) | 50°C | 6.5 hours | 88% |
| 9 | cyclohexyl acrylate (0.54 mol) | paraformaldehyde (1.1 mol) | DABCO (0.65 mol) | 324 ml (18 mol) | DMAc (10 mol) | sodium chloride (2.16 mol) | 50°C | 4 hours | 90% |
| 10 | methyl acrylate (0.54 mol) | 35% methanol aqueous solution of formaldehyde (1.0 mol) | DABCO (0.50 mol) | 360 ml (20 mol) | DMAc (8.7 mol) | lithium chloride (1.0 mol) | 50°C | 6.5 hours | 85% |
| 11 | methyl acrylate (0.54 mol) | paraformaldehyde (1.1 mol) | DABCO (0.65 mol) | 324 ml (18 mol) | NMP (8.7 mol) | sodium (2.16 mol) | 50°C | 4 hours | 87% |
| 12 | cyclohexyl acrylate (0.54 mol) | paraformaldehyde (1.1 mol) | DABCO (0.65 mol) | 324 ml (18 mol) | DMAc (9.0 mol) | lithium chloride (2.16 mol) | 70°C | 3 hours | 83% |
| 13 | cyclohexyl acrylate (0.54 mol) | paraformaldehyde (1.1 mol) | DABCO (0.65 mol) | 324 ml (18 mol) | acetonitrile (10 mol) | sodium chloride (1.0 mol) | 50°C | 6.5 hours | 85% |
| 14 | methyl acrylate (0.54 mol) | paraformaldehyde (1.1 mol) | DABCO (0.65 mol) | 360 ml (20 mol) | acetonitrile (10 mol) | calcium chloride (1.0 mol) | 50°C | 6.5 hours | 83% |
| 15 | ethyl acrylate (0.54 mol) | paraformaldehyde (1.1 mol) | DABCO (0.65 mol) | 324 ml (18 mol) | DMAc (8.7 mol) | potassium chloride (2.16 mol) | 50°C | hours | 83% |
| 16 | methyl acrylate (0.54 mol) | paraformaldehyde (1.1 mol) | DABCO (0.65 mol) | 360 ml (20 mol) | acetonitrile (10 mol) | potassium chloride (2.16 mol) | 50°C | 6 hours | 82% |
| Comparative Example 1 | ethyl acrylate (0.54 mol) | paraformaldehyde (1.1 mol) | DABCO (0.50 mol) | 324 ml (18 mol) | DMAc (8.7 mol) | not added | 50°C | 10 hours | 74% |

The production process of the present invention ensures that the reaction rate can be enhanced and at the same time, the yield of the objective hydroxyl group-containing vinyl compound can be increased. Therefore, this production process is industrially advantageous.

The entire disclosure of each and every foreign patent application from which the benefit of foreign priority has been claimed in the present application is incorporated herein by reference, as if fully set forth.

## Claims

1. A process for producing a hydroxyl group-containing vinyl compound, the process comprising:
reacting an acrylate compound and an aldehyde-based compound in a presence of a tertiary amine compound and water in a reaction solution to produce a hydroxyl group-containing vinyl compound,
wherein the reaction is performed by adding an inorganic salt of a Group 1 or 2 metal of Periodic Table to the reaction solution.

2. The process for producing a hydroxyl group-containing vinyl compound according to claim 1,
wherein the inorganic salt is a lithium salt, a sodium salt or a potassium salt.

3. The process for producing a hydroxyl group-containing vinyl compound according to claim 1,
wherein a molar ratio (inorganic salt/acrylate compound) between the inorganic salt and the acrylate compound is from 0.2 to 10.

4. The process for producing a hydroxyl group-containing vinyl compound according to claim 1,
wherein a molar ratio (acrylate compound/aldehyde-based compound) between the acrylate compound and the aldehyde-based compound is 2 or less.

5. The process for producing a hydroxyl group-containing vinyl compound according to claim 1,
wherein a molar ratio (water/aldehyde-based compound) between the water and the aldehyde-based compound is from 7 to 20.

6. The process for producing a hydroxyl group-containing vinyl compound according to claim 1,
wherein the aldehyde-based compound is paraformaldehyde.

7. The process for producing a hydroxyl group-containing vinyl compound according to claim 1,
wherein the reaction is performed by further adding to the reaction solution an aprotic polar solvent in an amount not allowing the reaction solution to form a two-phase system at initiation of the reaction.

8. The process for producing a hydroxyl group-containing vinyl compound according to claim 1,
wherein the inorganic salt is added to the reaction solution before the reaction starts.
